Europäisches Patentamt

European Patent Office

Office européen des brevets

(19)

(11) Numéro de publication: **0 023 459**
**B1**

(12) **FASCICULE DE BREVET EUROPEEN**

(45) Date de publication du fascicule du brevet: **28.07.82**

(21) Numéro de dépôt: **80401102.1**

(22) Date de dépôt: **24.07.80**

(51) Int. Cl.³: **C 07 C 59/52,**
**C 07 C 59/56,**
**C 07 C 59/64,**
**C 07 C 51/367**

(54) Procédé de préparation d'acides hydroxyarylglycoliques racémiques.

(30) Priorité: **25.07.79 FR 7919171**

(43) Date de publication de la demande:
**04.02.81 Bulletin 81/5**

(45) Mention de la délivrance du brevet:
**28.07.82 Bulletin 82/30**

(84) Etats contractants désignés:
**BE CH DE GB IT LI NL**

(56) Documents cités:
**EP - A - 0 003 825**
**BE - A - 867 287**
**DE - A - 2 820 856**
**FR - A - 2 379 501**
**FR - A - 2 426 670**
**FR - A - 2 427 322**
**FR - A - 2 440 350**
**US - A - 4 198 523**

**CHEMICAL ABSTRACTS, vol. 91, no. 23, 3
décembre 1979, page 615, colonne 2, réf.:
193005r Columbus, Ohio, US.**

(73) Titulaire: **SOCIETE FRANCAISE HOECHST Société
anonyme dite:**
**3, avenue du Général de Gaulle**
**F-92800 Puteaux (FR)**

(72) Inventeur: **Schouteeten, Alain**
**17, rue de Normandie**
**F-95460 Ezanville (FR)**
Inventeur: **Christidis, Yani**
**12, rue de Constantinople**
**F-75008 Paris (FR)**

(74) Mandataire: **Rinuy, Guy et al,**
**14, Avenue de la Grande Armée**
**F-75017 Paris (FR)**

(56) Documents cités:
**CHEMICAL ABSTRACTS, vol. 92, no. 3, 21
janvier 1980, page 661, colonne 2, réf.: 22263q
Columbus, Ohio, US.**
**CHEMICAL ABSTRACTS, vol. 91, no. 19, 5
novembre 1979, page 612, colonne 2, réf.
157449e Columbus, Ohio, US.**
**CHEMICAL ABSTRACTS, vol. 91, no. 17, 22
octobre 1979, page 631, colonne 1, réf.
140572s Columbus, Ohio, US.**
**CHEMICAL ABSTRACTS, vol. 83, no. 7, 18—
08—1975, page 466, colonne 1, réf. 58435q
Columbus, Ohio, US.**

Courier Press, Leamington Spa, England.

# 0 023 459

Procédé de préparation d'acides hydroxyarylglycoliques racémiques

La présente invention concerne un nouveau procédé de préparation d'acides ortho ou parahydroxyarylglycoliques racémiques et les produits nouveaux en résultant.

Les acides hydroxyarylglycoliques racémiques sont des matières premières précieuses en synthèse organique depuis que les travaux de P. Hébert, Bull. Soc. Chim. France, 1920, 27 (4ème série). 45—55 ont confirmé leur dégradation en dérivé carbonylé par oxydation decarboxylante.

Il est connu dans l'Art antérieur d'accéder aux acides hydroxyarylglycoliques racémiques à partir d'un dérivé aromatique phénolique en milieu aqueux alcalin soit par condensation de l'acide glyoxylique selon le brevet des Etats-Unis d'Amérique No 2062205 soit par condensation du chloral suivie d'une hydrolyse de l'hydroxyaryltrichlorométhylcarbinol formé selon le brevet autrichien No 141159.

On sait que l'acide glyoxylique en milieu aqueux alcalin est peu stable et qu'il se dismute quantitativement à chaud en acides oxalique et glycolique par réaction de Cannizzaro (BOETTINGER, Ber., 1880, 13 1931). Pour cette raison, les réactions faisant intervenir l'acide glyoxylique en milieu aqueux alcalin sont réalisées à température inférieure ou égale à la température ambiante. Ainsi, dans le procédé décrit dans le brevet français No 2 132 364, la condensation de l'acide glyoxylique sur le phénol en excès dissous dans la soude aqueuse à 10% est réalisée entre 15 et 25°C durant 36 heures. Dans le brevet allemand No 621 567, on abandonne plusieurs jours à la température ambiante une solution aqueuse alcaline de phénol et d'acide glyoxylique pour obtenir l'acide parahydroxymandélique racémique. J. GOODMAN et Coll., Biochem. Biophys. Acta, 1968, 156, 364.67, condensent l'acide glyoxylique sur le gaïacol en 68 heures à 25°C et J. KAMLET, brevet des Etats-Unis d'Amérique No 2 640 083 opère en 48 heures à 15—20°C pour préparer l'acide hydroxy-4 méthoxy-3 mandélique racémique. Récemment, le brevet belge No 867 287 décrit un procédé d'obtention d'un sel de métal alcalin de l'acide parahydroxymandélique monohydraté solide par condensation de l'acide glyoxylique sur le phénol en excès en solution aqueuse alcaline à une température légèrement supérieure à la température ambiante soit en 18 heures de 30 à 35°C (exemple 1), soit en 5 heures à 35°C (exemple 2), soit enfin en 8 heures à 35°C (exemple 3).

La demande de brevet européen No 0 003 825 et la demande de brevet japonais JP Kokai No 79—76542 enseignent un procédé de préparation de l'acide chloro-3 hydroxy-4 mandélique à partir de l'o-chlorophénol et de l'acide glyoxylique; selon les exemples donnés cette condensation est effectuée en milieu aqueux alcalin soit 30°C durant 24 heures soit à 50°C durant 6 heures.

Selon A. I. FATIADI et R. SCHAFFER, J. Research Nat. Bur. Stand., Sect. A, 1972n 78 A, 411—12, toute élévation de température est néfaste au rendement de condensation de l'acide glyoxylique sur le gaïacol pour accéder à l'acide hydroxy-4 méthoxy-3 mandélique racémique et ces auteurs recommandent de réaliser cette condensation en introduisant en 4 heures l'acide glyoxylique dans une solution aqueuse alcaline et refroidie vers 0°C de gaïacol, puis d'abandonner le milieu réactionnel 20 heures de 0 à 20°C. Pour la fabrication de l'acide dihydroxy-3,4 mandélique racémique la Société NIPPON SYNTHETIC CHEMICAL INDUSTRY Co. Ltd, dans la demande de brevet japonais 75—29522 (C.A. Vol. 83 58435a) préconise d'effectuer la condensation de l'acide glyoxylique sur le pyrocatéchol en 2 heures à 5—8°C, puis en 24 heures à 10—15°C et enfin 24 heures à la température ambiante.

Dans sa demande de brevet français 78—31123 la Demanderesse revendique un procédé de fabrication de l'acide para-hydroxymandélique racémique par condensation dans l'eau en présence d'un agent alcalin à une température comprise entre 30 et 100°C de l'acide glyoxylique sur un excès de phénol.

La Demanderesse a maintenant trouvé un procédé rapide de fabrication d'acides ortho- ou para-hydroxyarylglycolique racémique en chauffant progressivement jusqu'à une température comprise entre 85 et 100°C, en milieu aqueux alcalin, l'acide glyoxylique sur un excès ou non de dérivé aromatique phénolique autre que le phénol et l'orthochlorophénol possédant au moins un proton sur un des sommets ortho ou para par rapport à la fonction phénol.

Cette condensation n'est pas régiosélective par suite de la résonance de l'anion phénolate communiquant aux sommets ortho et para un certain caractère de carbanion.

Malgré la non régiosélectivité de cette condensation, elle fournit généralement d'une manière majoritaire l'isomère para par rapport au groupement phénolique sauf si cette position est soit déjà substituée comme dans le cas des phénols para-substitués soit encombrée stériquement par la présence d'un groupement volumineux sur un des sommets méta du phénol de départ comme dans le cas de l'éthyl-3 phénol soit enfin désactivée par les effets électroniques de substituants divers du phénol de départ.

Le pourcentage des isomères présents en fin de traitement dans le produit brut de la réaction peut être évalué soit par la spectroscopie de résonance magnétique nucléaire du proton soit par l'analyse potentiométrique en milieu non aqueux à l'aide de l'hydroxyde de tétrabutylammonium. En effet, en RMN, le proton méthine en position benzylique des acides ortho-hydroxyarylglycoliques résonne à champs plus faible que son homologue des acides parahydroxyarylglycoliques. Par dosage de la fonction carboxylique en milieu non aqueux, on observe généralement que les acides ortho-hydroxyarylglycoliques sont plus acides que les isomères para correspondants. De plus, par dosage en

2

**0 023 459**

milieu aqueux de la fonction phénol, on remarque dans de très nombreux cas qu'elle ne peut être dosée lorsqu'elle est en position ortho de la chaîne latérale acide hydroxy-2 acétique.

Les dérivés aromatiques phénoliques utilisables comme produits de départ sont par exemple le gaïacol, l'éthoxy-2 phénol (guéthol), le pyrocatéchol, l'ortho-crésol, le para-crésol, l'orthotertiobutyl-phénol, le paratertiobutylphénol, l'ortho-chlorophénol, le méta-éthyl-phénol, le métafluorophénol, le diméthoxy-2,6 phénol, le $\beta$-naphtol et analogues.

Selon l'invention, on chauffe pendant quelques dizaines de minutes et progressivement jusqu'à une température comprise entre 85 et 100°C, en milieu aqueux alcalin, l'acide glyoxylique sur un excès ou non de dérivé aromatique phénolique, puis après neutralisation du milieu réactionnel à pH de l'ordre de 6,5, on élimine le dérivé phénolique non transformé par extraction avec un solvant organique non miscible ou peu miscible à l'eau, ensuite on acidifie à pH de l'ordre de 0,5 la phase aqueuse et on extrait le produit cherché avec un solvant non miscible ou peu miscible à l'eau. On isole ainsi, après élimination du solvant d'extraction et recristallisation du résidu, l'acide ortho ou para-hydroxyarylglycolique racémique cherché pur.

Les exemples suivants sont donnés à titre purement illustratif et nullement limitatif de l'invention.

Exemple 1

On chauffe en 45 minutes de 35 à 85°C, sous agitation et en atmosphère d'azote, une solution obtenue en dissolvant:

— 1,5 mole (186,2 g) de gaïacol;
— 0,5 mole (74 g) d'acide glyoxylique à 50% dans l'eau;
— 1,125 mole (45 g) d'hydroxyde de sodium en pastilles dans 900 g d'eau.

Après refroidissement de 85 à 30°C, en 20 minutes, on acidifie le milieu réactionnel à pH 6,5 avec de l'acide chlorhydrique concentré (d=1,18) puis on extrait le gaïacol non transformé à l'acétate d'éthyle. On isole ainsi, après élimination du solvant d'extraction, 1,04 mole (129,1 g) de gaïacol. La phase aqueuse résiduelle acidifiée ensuite à pH 0,5 avec de l'acide chlorhydrique concentré est lavée à l'acétate d'éthyle, pour extraire le produit cherché.

Après élimination sous vide de l'acétate d'éthyle, on recueille 80 g de produit cristallisé possédant un point de fusion instantané de 116°C. Ce produit est recristallisé dans 10 volumes de nitrométhane; on isole ainsi après séchage sous vide à 80°C à poids constant, 66,3 g (0,33 mole) d'acide hydroxy-4 méthoxy-3 mandélique racémique possédant un point de fusion de 132±1°C, soit un rendement de 66% par rapport à l'acide glyoxylique mis en oeuvre (littérature: F=133°C, J.A.F. GARDNER et Coll., J. Amer. Chem. Soc., 1944, *66*, 607,)

RMN (acétone d$_6$)
$\delta$=3,75 ppm s 3H (OCH$_3$)
$\delta$=5,05 ppm s 1H (H benzylique)
$\delta$=6,65—7,05 ppm m 3H (H aromatiques)

Exemple 2

On opère selon l'exemple 1, mais on remplace le gaïacol par l'ortho-éthoxyphénol 1,5 mole (207,2 g). En fin de traitement, on récupère 142,3 g (1,03 mole) d'orthoéthoxyphénol et on recueille 83,5 g de produit cristallisé possédant un point de fusion de 121±1°C. Après recristallisation dans 7 volumes de nitrométhane et séchage sous vide à 80°C à poids constant, on isole 72,37 g (0,341 mole) d'acide éthoxy-3 hydroxy-4 mandélique racémique possédant un point de fusion de 126±1°C, soit un rendement de 68,2% par rapport à l'acide glyoxylique mis en oeuvre (littérature F=104—105°C, hydrate avec 1 mole H$_2$O, P. P. SHORYGIN et Coll., Sintezy Dushistykh Veshchestv, Sbornik Staiei, *1939*, 7—13; C.A. *42*, 3267).

Microanalyse
$C_{10}H_{12}O_5$=212,20

| | C% | H% | O% |
|---|---|---|---|
| calculés | 56,60 | 5,70 | 37,70 |
| trouvés | 56,20 | 5,6 | |

RMN (acétone d$_6$)
$\delta$=1,33 ppm t 3H (H méthyle) J=7Hz
$\delta$=4,03 ppm q 2H (H—OCH$_2$) J=7Hz
$\delta$=5,22 ppm s 1H (H benzylique)
$\delta$=6,6—6,8 ppm m 3H (H aromatique)

Exemple 3

On opère selon l'exemple 1, mais on remplace le gaïacol par le pyrocatéchol 1,5 mole (165,2 g). En fin de traitement, on récupère 1,02 mole (112,3 g) de pyrocatéchol non transformé et on recueille

3

88,2 g de produit cristallisé possédant un point de fusion de 124°C. Après recristallisation dans 30 volumes de nitrométhane et séchage sous vide à 80°C à poids constant, on isole 52,9 g (0,287 mole) d'acide dihydroxy-3,4 mandélique racémique possédant un point de fusion de 138±1°C soit un rendement de 57,4% par rapport à l'acide glyoxylique mis en oeuvre (littérature: F=137°C, K. N. F. Shaw et Coll., J. Org. Chem. 1958, 23, 31).

R.M.N. (acétone $d_6$)
$\delta$=4,93 ppm s 1H (H benzylique)
$\delta$=6,7—6,8 ppm m 3H (aromatique)

Exemple 4

On opère selon l'exemple 1, mais on remplace le gaïacol par l'ortho-crésol 1,5 mole (162,2 g). En fin de traitement, on récupère 0,97 mole (105 g) d'ortho-crésol non transformé et on recueille 120 g de produit cristallisé possédant un point de fusion de 105°C. Après recristallisation dans 120 volumes de dichloroéthane et séchage sous vide à 80°C à poids constant, on isole 68 g (0,373 mole) d'acide hydroxy-4 méthyl-3 mandélique possédant un point de fusion de 114±1°C soit un rendement de 74,6% par rapport à l'acide glyoxylique mis en oeuvre.

Microanalyse
$C_9H_{10}O_4$=182,17

| | C% | H% | O% |
|---|---|---|---|
| calculés | 59,33 | 5,53 | 35,13 |
| trouvés | 59,2 | 5,5 | |

RMN du proton (acétone $d_6$)
$\delta$=2,15 ppm s, 3H ($CH_3$)
$\delta$=5,05 ppm s, 1H (H benzylique)
$\delta$=6,55—7,15 ppm m, 3H (aromatique)

A la connaissance de la Demanderesse ce produit n'est pas décrit dans la littérature. L'acide hydroxy-4 méthyl-3 mandélique est cité dans la littérature: P. KÖHLER et H. BAUFELD, Aerztlolal, 1964, 10 (7), 224—25; C.A. 1964, 61, 16415a, mais l'examen de la publication indique qu'il s'agit de l'acide méthoxy-3 hydroxy-4 mandélique ce qui confirme par ailleurs le résumé effectué par les auteurs en fin de publication.

Exemple 5

On opère selon l'exemple 1, mais on remplace le gaïacol par 1,5 mole (225,3 g) d'ortho-tertio-butylphénol. En fin de traitement, on récupère 1,29 mole d'ortho-tertiobutylphénol non transformé et on recueille 21,3 g de produit cristallisé qui fournissent après recristallisation dans 4 volumes de nitrométhane et séchage sous vide à 80°C à poids constant 12,8 g (0,057 mole) d'acide hydroxy-4 tertiobutyl-3 mandélique racémique possédant un point de fusion de 158±1°C, soit un rendement de 11,5% par rapport à l'acide glyoxylique mis en oeuvre.

Dosage potentiométrique

— fonction carboxylique 4,44 méq/g (théorie 4,46 méq/g).

RMN (acétone $d_6$)
$\delta$=1,4 ppm s 9H (tert.Bu)
$\delta$=5,08 ppm s 1H (benzylique)
$\delta$=6,7 ppm d 1H ($H_5$ aromatique) $JH_5$—$H_6$=8Hz
$\delta$=7,12 ppm q 1H ($H_6$ aromatique) $JH_6$—$H_5$=8Hz $JH_6H_2$=2Hz
$\delta$=7,4 ppm d 1H ($H_2$ aromatique) $JH_2$—$H_6$=2Hz

A la connaissance de la Demanderesse, ce produit n'est pas décrit dans la littérature.

Exemple 6

On opère selon l'exemple 1, mais on remplace le gaïacol par 1,5 mole (225,3 g) de paratertiobutylphénol. En fin de traitement, on récupère 1,35 mole (202,8 g) et on recueille 28 g de produit cristallisé qui fournissent après recristallisation dans 3 volumes de dichloro-1,2 éthane, 16,15 g (0,072 mole) d'acide hydroxy-2 tertiobutyl-5 mandéliquie racémique possédant un point de fusion de 119±1°C soit un rendement de 14,4% par rapport à l'acide glyoxylique mis en oeuvre.

Microanalyse
$C_{12}H_{16}O_4$=224,25

| | C% | H% | O% |
|---|---|---|---|
| calculés | 64,27 | 7,19 | 28,54 |
| trouvés | 64,3 | 7,15 | |

RMN (acétone d$_6$)

$\delta$=1,23 ppm s 9H (tertioBu)

$\delta$=5,38 ppm s 1H (H benzylique)

$\delta$=6,7 ppm d 1H (H$_3$ aromatique) JH$_3$H$_4$=8,5Hz

$\delta$=7,13 ppm q 1H (H$_4$ aromatique) JH$_4$H$_3$=8,5H$_3$ JH$_4$H$_6$=2H$_3$

$\delta$=7,3 ppm d 1H (H$_6$ aromatique) JH$_6$H$_4$=2Hz

A la connaissance de la Demanderesse, ce produit n'est pas décrit dans la littérature.

Exemple 7

On opère selon l'exemple 1, mais on remplace le gaïacol par 1,5 mole (192,8 g) d'ortho-chloro-phénol. En fin de traitement, on récupère 1,07 mole (137,6 g) d'orthochlorophénol non transformé et on recueille 82,4 g de produit cristallisé (F=130—138°C) qui fournissent après recristallisation dans 10 volumes de nitrométhane et séchage à poids constant sous vide à 80°C, 68,2 g (0,337 mole) d'acide chloro-3 hydroxy-4 mandélique racémique possédant un point de fusion de 146±1°C, soit un rendement de 67,4% par rapport à l'acide glyoxylique mis en oeuvre.

Microanalyse

|  | | C% | H% | Cl% | O% |
|---|---|---|---|---|---|
| C$_8$H$_7$Cl O$_4$=202,6 | calculés | 47,42 | 3,48 | 17,50 | 31,59 |
|  | trouvés | 47,6 | 3,6 | 17,4 | |

RMN (acétone d$_6$)

$\delta$=5,13 ppm s 1H (H benzylique)

$\delta$=6,93 ppm d 1H (H$_5$ aromatique) JH$_5$H$_6$=9Hz

$\delta$=7,27 ppm q 1H (H$_6$ aromatique) JH$_6$H$_5$=9Hz JH$_6$H$_2$=2Hz

$\delta$=7,43 ppm d 1H (H$_2$ aromatique) JH$_2$H$_6$=2Hz

A la connaissance de la Demanderesse, ce produit n'est pas décrit dans la littérature.

Exemple 8

On opère selon l'exemple 1, mais on remplace le gaïacol par 1,5 mole (186,2 g) d'éther monométhylique de l'hydroquinone (para-méthoxyphénol). En fin de traitement, on récupère 1,09 mole (135,3 g) d'éther monométhylique de l'hydroquinone non transformé et on recueille 81 g de produit cristallisé, F=129±1°C, qui fournissent après recristallisation dans 10 volumes de nitrométhane et séchage à 80°C sous vide à poids constant 65,6 g (0,331 mole) d'acide hydroxy-2 méthoxy-5 mandélique racémique possédant un point de fusion de 135—136°C soit un rendement de 66,2% par rapport à l'acide glyoxylique mis en oeuvre. (F=135°C–Th. Koppe et Th Witoszynskyj, Arch. Pharm. 1975, 308,344).

Microanalyse

|  | | C% | H% | O% |
|---|---|---|---|---|
| C$_9$H$_{10}$O$_5$=198,17 | calculés | 54,54 | 5,08 | 40,37 |
|  | trouvés | 54,6 | 5,05 | |

RMN (diméthylsulfoxyde d$_6$)

$\delta$=3,6 ppm s 3H (—OCH$_3$)

$\delta$=5,18 ppm s 1H (H benzylique)

$\delta$=6,6—6,8 ppm m 3H (H aromatique)

Exemple 9

On opère selon l'exemple 1, mais on remplace le gaïacol par 1,5 mole (162,2 g) de para-crésol. En fin de traitement, on récupère 1,12 mole (121,1 g) de para-crésol non transformé et on recueille 78 g de produit cristallisé qui fournissent après recristallisation dans 2 volumes de nitrométhane et séchage sous vide à poids constant à 80°C, 42,5 g (0,233 mole) d'acide hydroxy-2 méthyl-5 mandélique racémique possédant un point de fusion de 104±1°C soit un rendement de 46,6% par rapport à l'acide glyoxylique mis en oeuvre.

Microanalyse

|  | | C% | H% | O% |
|---|---|---|---|---|
| C$_9$H$_{10}$O$_4$=182,17 | calculés | 59,33 | 5,53 | 35,13 |
|  | trouvés | 59,3 | 5,6 | |

RMN du proton (acétone d$_6$)

$\delta$=2,18 ppm s 3H (CH$_3$)

**0 023 459**

$\delta$=5,37 ppm s 1H (H benzylique)
$\delta$=6,65 ppm d 1H (H$_3$ aromatique) JH$_3$H$_4$=8Hz
$\delta$=6,9 ppm q 1H (H$_4$ aromatique) JH$_4$H$_3$=8Hz JH$_4$H$_6$=1,5Hz
$\delta$=7,03 ppm d 1H (H$_6$ aromatique) JH$_6$H$_4$=1,5Hz

Dosage par potentiométrie
Fonction phénol 5,44 méq/g
Fonction carboxyle 5,45 méq/g                              (théorie: 5,5 méq/g)

A la connaissance de la Demanderesse, ce produit n'est pas décrit dans la littérature. Toutefois son sel de potassium en solution aqueuse a été obtenu intermédiairement par F. BOEDECKER, (brevet allemand 621567) au cours de la préparation de l'hydroxy-2 méthyl-5 benzaldehyde à partir du para-cresol.

Exemple 10

On opère selon l'exemple 1, mais on remplace le gaïacol par 1,5 mole (183,2 g) de méta-éthyl-phénol. En fin de traitement, on récupère 1,1 mole (134,4 g) de méta-éthyphénol et on recueille 95 g de produit brut. Ce produit brut est analysé par RMN du proton en solution dans le diméthylsulfoxyde deutéré et les signaux du proton en position benzylique sont intégrés. Les spectres indiquent que ce produit brut est constitué de 30% d'acide éthyl-2 hydroxy-4 mandélique racémique, isomère para, déplacement chimique du proton benzylique $\delta$=5 ppm et de 70% d'acide éthyl-4 hydroxy-2 mandélique racémique, isomère ortho, déplacement chimique du proton benzylique $\delta$=5,15 ppm.

Par recristallisation dans le nitrométhane, on isole l'acide éthyl-4 hydroxy-2 mandélique racémique possédant un point de fusion de 98±2°C.

Microanalyse
$C_{10}H_{12}O_4$=196,20

|  | C% | H% | O% |
|---|---|---|---|
| calculés | 61,21 | 6,17 | 32,62 |
| trouvés | 61,4 | 6,1 |  |

RMN (diméthylsulfoxyde d$_6$)
$\delta$=1,15 ppm t 3H (CH$_3$) J=7,5Hz
$\delta$=2,5 ppm q 2H (CH$_2$) J=7,5Hz
$\delta$=5,15 ppm s 1H (H benzylique)
$\delta$=6,55 ppm q 1H (H$_5$ aromatique) JH$_5$H$_6$=8Hz JH$_5$H$_3$=1Hz
$\delta$=6,6 ppm d 1H (H$_3$ aromatique) JH$_3$H$_5$=1Hz
$\delta$=7,05 ppm d 1H (H$_6$ aromatique) JH$_6$H$_5$=8Hz

A la connaissance de la Demanderesse, ce produit n'est pas connu dans la littérature.

Exemple 11

On opère selon l'exemple 1, mais on remplace le gaïacol par 1,5 mole (168,15 g) de méta-fluorophénol. En fin de traitement, on récupère 1,03 mole (115,5 g) de méta-fluorophénol et on recueille 91 g de produit cristallisé, F=127±2°C, qui fournissent après recristallisation dans 5 volumes de nitrométhane et séchage à poids constant à 80°C sous vide, 62,5 g (0,336 mole) d'acide fluoro-2 hydroxy-4 mandélique racémique, possédant un point de fusion de 144°C soit un rendement de 67,2% par rapport à l'acide glyoxylique mis en oeuvre.

Microanalyse
$C_8H_7F O_4$=186,14

|  | C% | H% | F% | O% |
|---|---|---|---|---|
| calculés | 51,61 | 3,79 | 10,21 | 34,38 |
| trouvés | 51,7 | 3,9 |  |  |

RMN (acétone d$_6$)
$\delta$=5,3 ppm s 1H (H benzylique)
$\delta$=6,47 ppm q 1H (H$_3$ aromatique) JH$_3$F=10Hz JH$_3$H$_5$=2Hz
$\delta$=6,57 ppm sext. 1H (H$_5$ aromatique) JH$_5$H$_3$=2Hz JH$_5$H$_6$=8Hz JH$_5$F=2Hz
$\delta$=7,21 ppm t 1H (H$_6$ aromatique) JH$_6$H$_5$=8Hz JH$_6$F=8Hz

A la connaissance de la Demanderesse, ce produit n'est pas décrit dans la littérature.

Exemple 12

On opère selon l'exemple 1, mais on remplace le gaïacol par 1,5 mole (231,2 g) de diméthoxy-2,6 phénol. En fin de traitement, on récupère 0,98 mole (151,1 g) de diméthoxy-2,6 phénol et on recueille 115 g de produit cristallisé, F=109°C, qui fournissent après recristallisation dans 5 volumes d'acétate d'éthyle et séchage sous vide à 80°C à poids constant 91,26 g (0,37 mole) d'acide diméthoxy-3,5 hydroxy-4 mandélique monohydraté racémique possédant un point de fusion de 109°C, soit un rendement de 74% par rapport à l'acide glyoxylique mis en oeuvre.

Microanalyse

$C_{10}H_{12}O_6,H_2O=246,22$

| | | C% | H% | O% | H2O* |
|---|---|---|---|---|---|
| | calculés | 48,78 | 5,73 | 45,49 | 7,32 |
| | trouvés | 49,0 | 5,7 | | 7,1 |

*déterminés par la méthode de K. Fischer

RMN (acétone $d_6$)
$\delta=3,72$ ppm s 6H (OCH$_3$)
$\delta=4,97$ ppm s 1H (H benzylique)
$\delta=6,67$ ppm s 2H (H aromatique)

A la connaissance de la Demanderesse, ce produit n'est pas décrit dans la littérature.

Exemple 13

On opère selon l'exemple 1, mais on remplace le gaïacol par 1,5 mole de $\beta$-naphtol (216,2 g). En fin de traitement, on récupère 1,1 mole (158,6 g) de $\beta$-naphtol et on recueille 91,5 g de produit cristallisé, F=126°C, qui fournissent après recristallisation dans 7 volumes de nitrométhane et séchage à 80°C sous vide à poids constant 24,2 g (0,111 mole) d'acide (hydroxy-1 naphtyl-2) glycolique racémique possédant un point de fusion de 126±1°C soit un rendement de 22,2% par rapport à l'acide glyoxylique mis en oeuvre.

Microanalyse

$C_{12}H_{10}O_4=218,20$

| | | C% | H% | O% |
|---|---|---|---|---|
| | calculés | 66,05 | 4,62 | 29,33 |
| | trouvés | 66,1 | 4,6 | |

RMN (diméthylsulfoxyde $d_6$)
$\delta=5,9$ ppm s 1H (H benzylique)
$\delta=6,98$—8,1 ppm m 6H (aromatique)

A la connaissance de la Demanderesse, ce produit n'est pas décrit dans la littérature.

Il va de soi que la présente invention n'a été décrite qu'à titre purement explicatif et nullement limitatif et que toute modification utile pourra y être apportée sans sortir de son cadre tel que défini par les revendications ci-après.

**Revendications**

1. Procédé de fabrication d'acides ortho- ou parahydroxyarylglycoliques racémiques par condensation en milieu aqueux alcalin de l'acide glyoxylique sur un excès ou non de dérivé aromatique phénolique autre que le phénol et l'orthochlorophénol possédant au moins un proton sur un des sommets ortho ou para par rapport à la fonction phénol caractérisé par le fait que cette condensation est effectuée en chauffant progressivement le milieu réactionnel jusqu'à une température comprise entre 85 et 100°C.

2. Procédé selon la revendication 1, caractérisé par le fait que la condensation s'effectue en chauffant le milieu réactionnel progressivement jusqu'à 85°C.

3. Procédé selon la revendication 1 ou 2 caractérisé par le fait que la condensation est effectuée entre 30 et 70 minutes.

**Patentansprüche**

1. Verfahren zur Herstellung von razimischen ortho-oder para-Hydroxyarylglycolsäuren durch Kondensation von Glyoxylsäure in wässrigem alkalischen Medium mit, ggf. einem Überschuß, von aromatischen Phenolderivaten ausgenommen Phenol und Orthochlorphenol, die mindestens ein Proton an der ortho- oder para-Stellung, bezogen auf die Phenolfunktion aufweisen, dadurch gekennzeichnet, daß diese Kondensation durch allmähliches Erhitzen des Reaktionsmediums bis zu einer Temperatur zwischen 85 und 100°C durchgeführt wird.

2. Verfahren nach Anspruch 1, dadurch gekennzeichnet, daß die Kondensation durchgeführt wird, indem man das Reaktionsmedium allmählich auf 85°C erhitzt.

**0 023 459**

3. Verfahren nach Anspruch 1 oder 2, dadurch gekennzeichnet, daß man die Kondensation 30 bis 70 Minuten lang durchführt.

**Claims**

1. A process for preparing racemic ortho or para hydroxyaryl-glycolic acids by condensing, in an alkaline aqueous medium, glyoxylic acid on an excess or without an excess of aromatic phenolic derivative other than phenol and orthochlorophenol, possessing at least one proton, on one of the ortho or para positions, with respect to the phenol function, characterized by carrying out condensation by progressively heating the reactional medium up to a temperature of between 85 and 100°C.

2. A process as in claim 1, characterized by effecting condensation by heating the reactional medium progressively up to 85°C.

3. A process as in claim 1 or 2, characterized by effecting condensation in 30 to 70 minutes.